Europäisches Patentamt

European Patent Office

Office européen des brevets

(19)

(11)  EP 0 973 494 B1

(12)  **FASCICULE DE BREVET EUROPEEN**

(45)  Date de publication et mention
de la délivrance du brevet:
**01.06.2005  Bulletin 2005/22**

(51)  Int Cl.⁷: $A61K\ 7/48$, $A61K\ 35/78$

(21)  Numéro de dépôt: **98903078.8**

(22)  Date de dépôt: **16.01.1998**

(86)  Numéro de dépôt international:
**PCT/FR1998/000084**

(87)  Numéro de publication internationale:
**WO 1998/031336 (23.07.1998 Gazette 1998/29)**

(54)  **UTILISATION D'UN EXTRAIT DU GENRE ADANSONIA**

VERWENDUNG EINES ADANSONIA-GATTUNG EXTRAKTES

USE OF AN EXTRACT OF THE GENUS ADANSONIA

(84)  Etats contractants désignés:
**AT BE CH DE DK ES FI FR GB GR IE IT LI LU MC
NL PT SE**

(30)  Priorité:  **20.01.1997  FR 9700709**

(43)  Date de publication de la demande:
**26.01.2000  Bulletin 2000/04**

(73)  Titulaire: **Cognis France S.A.
31360 Saint Martory (FR)**

(72)  Inventeur: **PAULY, Gilles
F-54000 Nancy (FR)**

(74)  Mandataire: **Nuss, Pierre et al
Cabinet Nuss
10, rue Jacques Kablé
67080 Strasbourg Cédex (FR)**

(56)  Documents cités:
**EP-A- 0 781 545**

- **WOODRUFF: "trough the natural ingredients
  maze" MANUFACTURING CHEMIST, vol. 65, no.
  10, octobre 1994, LONDON, pages 23-25,
  XP002037519**
- **STN, Serveur de Bases de Données,
  XP002037520**
- **STN, Serveur de Bases de Données,
  XP002037521**
- **STN, Serveur de Bases de Données,
  XP002037522**

EP 0 973 494 B1

**Description**

La présente invention concerne le domaine de la cosmétologie et de la dermatologie, et a pour objet l'utilisation pour des applications cosmétiques, dermatologiques ou pharmaceutiques, d'un extrait de plante du genre Adansonia, plus particulièrement de l'espèce Adansonia Digitata (Baobab), ainsi qu'un produit ou une composition cosmétique et/ou pharmaceutique pour la peau et/ou les phanères comportant un tel extrait.

Le Baobab (Adansonia Digitata) est un arbre caduque, originaire des parties arides d'Afrique centrale et apparaît dans la plupart des pays des tropiques, principalement comme composant des forêts secondaires.

L'origine des plantes du genre Adansonia se situe probablement à Madagascar où plusieurs espèces endémiques ont été décrites et où Adansonia digitata existe également. D'autres espèces du genre précité ont été relevées en Afrique orientale et en Australie.

Les différentes parties constitutives du Baobab étaient et sont encore utilisées et exploitées en Afrique, soit du point de vue économique (l'écorce pour la fabrication de cordages et de papier, la pulpe comme coagulant du caoutchouc et les racines comme matière colorante rouge), soit du point de vue alimentaire (plus particulièrement les graines et les jeunes feuilles) ou même du point de vue médical (l'écorce présente des propriétés astringentes, diaphorétiques et même fébrifuges ; la pulpe et les graines présentent des propriétés antidysentériques et anti-inflammatoires ; les feuilles sont utilisées contre la transpiration, contre les troubles du rein et de la vessie et comme anti-asthmatique et émollient).

Il est connu que les feuilles contiennent notamment des mucilages qui gonflent en présence d'eau.

Les feuilles de Baobab (YAZZIE D et al., Journal of food composition and analysis, 1994, 7;3, 198-193 - GAIWE R et al., International Journal of crude drug research, 1989, 27, 2, 101-104) contiennent, en plus des mucilages, des sels minéraux, des protéines, des tannins catéchiques et des composés vitaminiques (Riboflavine, Thiamine, Vitamine C, Niacine) : un composé de nature flavonoïque a également été mis en évidence.

L'analyse de la composition en aminoacides indique que les protéines de feuilles de Baobab, qui représentent environ 10,6 % du poids sec des feuilles, contiennent des quantités intéressantes des acides aminés essentiels suivants : lysine, arginine, thréonine, tyrosine, phénylalanine, tryptophane, méthionine et cystéine.

Ces feuilles constituent quantitativement et qualitativement une bonne source de protéines alimentaires.

De plus, les feuilles de Baobab contiennent des quantités élevées de calcium (3,07 à 30 mg/g feuilles sèches) et des quantités substantielles de fer, de potassium, de magnésium, de manganèse, de molybdène, de phosphore et de zinc.

La teneur des feuilles en mucilages bruts extractibles varie et est de l'ordre de 9 à 12 % par rapport aux feuilles sèches, les constituants principaux de ces mucilages présentant des poids moléculaires supérieurs à 100 000.

Une interaction élevée entre les protéines et les polysaccharides est supposée.

D'après la littérature (WOOLFE M.L. et al, J. Sci. Fd Agric., 1977, 28, 519-529), la composition chimique des mucilages de feuilles de Baobab s'établit comme suit :

- 40,2 g d'acide galacturonique / 100 g de mucilages
- 39,1 g d'acide glucuronique / 100 g de mucilages
- 9,3 g de sucres neutres / 100 g de mucilages.

Ces sucres neutres étant le rhamnose, le galactose, le glucose et l'arabinose qui sont présents dans un rapport molaire de 0,6-1-0,44-0,15.

Les données ci-dessus indiquent une très forte proportion d'acides uroniques et peu de sucres neutres : ces mucilages ne présenteraient pas de composés pectiques ou d'unités du type pectique.

Par ailleurs, ces mucilages possèdent des propriétés rhéologiques intéressantes, leur viscosité diminuant avec une élévation de la température d'extraction.

Le document STN, Database, XP002037520, Fichier Medline, AN=95278136, Karlsruhe, Allemagne, fait état de l'utilisation d'un extrait de feuilles de plante Adansonia digitata, mais uniquement pour la préparation d'une composition topique pour lutter contre l'inflammation, la douleur et pour soigner les blessures.

Or, les inventeurs de la présente invention ont constaté, de manière inattendue et surprenante, que les extraits de plantes du genre Adansonia, et plus particulièrement les extraits enrichis en mucilages, présentaient des propriétés immédiates plus variées et quantitativement nettement plus importantes que celles des polysaccharides déjà utilisés en cosmétique ou en pharmacologie, ainsi que des effets à long terme et enfin une très bonne tolérance.

Ainsi, le principal objet de la présente invention consiste en l'utilisation ou l'application d'au moins un extrait de plante du genre Adansonia appartenant à la famille des Bombacaceaes telle que mentionnée dans la revendication 1.

Selon un mode de réalisation préférentiel de l'invention, l'extrait précité est obtenu à partir des feuilles fraîches ou séchées (par exemple réduites en poudre) de Baobab, l'extraction étant réalisée selon les techniques classiques d'extraction, telle que l'extraction à chaud ou à froid, par un solvant choisi dans le groupe formé par l'eau, les solutions aqueuses (neutres, acidifiées ou alcalinisées), les alcools et les mélanges de deux ou plusieurs des solvants précités.

Conformément à une première variante de réalisation de l'invention, l'extrait utilisé est un extrait total de feuilles de Baobab, contenant tous les ingrédients actifs contenus dans ladite feuille.

Cet extrait total peut être séché par les techniques connues de l'homme du métier telles que la lyophilisation ou l'atomisation.

Conformément à une seconde variante de réalisation de l'invention, il est possible de procéder à des opérations supplémentaires de purification (par exemple par précipitation dans des solvants organiques) permettant d'aboutir, d'une part, à un extrait consistant en un ou des mucilage(s) purifié(s) ou en un extrait riche en mucilage(s) obtenu(s) à partir des feuilles de Baobab, et/ou, d'autre part, à un extrait consistant en un co-produit de l'extraction et/ou de la purification de mucilages des feuilles de Baobab, ledit co-produit constituant une fraction directement exploitable et riche en flavonoïdes, sels minéraux, protéines, vitamines et/ou autres composés analogues, tels que notamment des tannins.

Il a également été constaté, de manière inattendue et surprenante, que lorsque le procédé d'extraction ou de purification comprenait une étape de traitement par une enzyme glycolytique du type β-glycosidase, le ou les mucilage(s) ou l'extrait riche en mucilage(s) résultant présentai(en)t une stabilité accrue en solution.

A titre illustratif et non limitatif, on décrira ci-après différents procédés d'obtention possibles d'un extrait de Baobab, notamment de mucilages, pouvant être utilisés dans le cadre de la présente invention.

Exemple 1 (production de l'extrait type 1)

2,20 Kg de feuilles d'Adansonia digitata sont broyées, dans un broyeur à couteaux et passées sur un tamis de 5mm, 2,00 kg de poudre de feuilles sont ainsi obtenus. Dans une cuve munie d'un agitateur, on introduit 20,00 kg d'eau distillée et on réalise ensuite successivement les opérations suivantes :

- monter la température à environ 70° C,
- introduire sous agitation les 2,00 kg de feuilles broyées et tamisées,
- monter la température à 90-95° C,
- extraire une heure sous agitation,
- refroidir,
- centrifuger pendant 10 minutes à 5 000 g,
- récupérer le surnageant (15,4 litres) qui présente un aspect visqueux, une couleur brune et comporte 2,6 % en poids d'extrait sec.

Des mucilages purifiés peuvent être obtenus en appliquant les traitements suivants au surnageant précité :

- précipiter les mucilages par addition du surnageant sous agitation violente, dans 0,6 volume d'éthanol absolu : formation de fibres qui s'enroulent autour de l'agitateur et surnageant hydroalcoolique de couleur brune,
- laisser deux heures dans ce milieu,
- récupérer les fibres, les essorer sur une toile filtrante,
- laver les fibres de polysaccharide dans 1,6 litres d'acétone (ce traitement pouvant être renouvelé),
- essorer les fibres, les étaler et les faire sécher à l'air libre puis à l'étuve à 50° C,
- broyer les mucilages secs au broyeur à couteaux.

Le poids de mucilages obtenus est de 168 grammes, soit un rendement de R = 8,4 % en poids par rapport aux feuilles broyées et un rendement d'environ 7,6 % en poids par rapport aux feuilles entières (avec tiges).

Exemple 2 (production de l'extrait type 2)

Dans une cuve munie d'un agitateur, on introduit 25,00 kg d'eau distillée et on réalise successivement les opérations suivantes :

- monter la température à environ 70° C,
- introduire sous agitation 2,00 kg de feuilles broyées et tamisées en agitant vigoureusement,
- monter la température à 90-95° C,
- extraire une heure sous agitation,
- refroidir,
- centrifuger pendant 10 minutes à 5 000 g,

- recueillir le surnageant (16,9 litres) qui présente un aspect visqueux, une couleur brune et comporte 2,3 % en poids d'extrait sec.
- placer le surnageant à 4° C jusqu'à l'hydrolyse.

Une détermination de la teneur en mucilages de la solution peut être réalisée par précipitation de 200 ml de surnageant dans un volume d'éthanol, lavage dans l'acétone et séchage du précipité obtenu. La concentration en mucilages dans l'extrait ainsi déterminée est de 9,15 g/l, soit un rendement en mucilages R = 7,7 % en poids par rapport à la poudre de feuilles broyées.

La purification des mucilages de l'extrait obtenu précédemment peut être effectuée en mettant en oeuvre les étapes suivantes :

- placer l'extrait visqueux dans un réacteur muni d'une électrode de pH,
- ajuster le pH de la solution à 5,0,
- monter la température à 52° C,
- ajouter à la solution une enzyme du type glucanase à la dose de 10 % par rapport aux mucilages déterminés par précipitation alcoolique,
- hydrolyser pendant 5 heures à une température de 50-55° C et un pH d'environ 5,0,
- inactiver l'enzyme en chauffant pendant 20 minutes à 100° C,
- refroidir à température ambiante,
- centrifuger,
- recueillir le surnageant (14,73 litres),
- précipiter les mucilages par addition du surnageant sous agitation violente dans 1 volume d'éthanol absolu,
- traiter le précipité selon l'exemple 1.

Le poids de mucilages récupérés est de 132,7 grammes, soit un rendement de R = 6,6 % en poids par rapport aux feuilles broyées initiales.

Exemple 3 (production de l'extrait type 3)

On filtre 1,9 litre de surnageant hydroalcoolique obtenu lors de la précipitation des mucilages de l'exemple 2 (extrait sec = 1,1 %) sur filtre clarifiant 0,5 μm.
Le rendement théorique en matière brute (en tenant compte de l'extrait sec et du volume total de surnageant hydroalcoolique) est de 7,76 % / poudre de feuilles.
Les traitements complémentaires suivants sont ensuite appliqués au surnageant filtré :

- évaporation de l'alcool de l'extrait à l'évaporateur rotatif (température 40° C),
- obtention de 0,91 litre de phase aqueuse avec un extrait sec de 2,2 %,
- addition éventuelle de 40 g d'adjuvant de déshydratation,
- atomisation ou lyophilisation,
- obtention de 39,9 g d'atomisat soit un rendement d'atomisation de 66,5 %.

La mise en évidence des composés flavonoïques dans le produit précité selon un procédé connu, (WAGNER H et al., Plant drug analysis, p. 172, Springer Verlag 1984), s'effectue en mettant en oeuvre les solvants de migration suivants : Acétate d'éthyle / Acide formique / Acide acétique glacial / Eau (100/11/11/27).
La révélation de flavonoïdes est réalisée par diphenyl-boric-acid-2-amino-ethyl ester à 1 % dans du méthanol / PEG 4000 à 5 % dans l'éthanol absolu.
La lecture s'effectue sous lampe U.V. 365 nm.
Elle met en évidence dans le co-produit un composé de couleur orangée après vaporisation du réactif et observation à 365 nm dont le Rf (0,39) est proche de celui de la rutine (0,40).
On observe également 4 taches de couleur orange, bleue et jaune dont les Rf sont compris entre 0,095 et 0,18.
La présente invention a également pour objet des produits ou des compositions cosmétiques et/ou pharmaceutiques pour la peau et/ou les phanères (cheveux, cils, ongles), caractérisés en ce qu'ils comportent entre 0,01 % et 50,00 % en poids d'un extrait de Baobab.
Préférentiellement, ces produits consistent en une composition traitante comportant entre 0,01 % et 20,00 % en poids d'extrait, notamment d'extrait de feuilles de Baobab.
Dans ces compositions ou produits de soins pour la peau et les phanères, les mucilages, protéines et sels minéraux extraits de plantes du genre Adansonia, et notamment de Baobab, ont été mis en oeuvre avantageusement en tant qu'actifs émollients, adoucissants, isolants, réparateurs, hydratants, apaisants, élastifiants, nutritifs et réparateurs des propriétés barrières.

Les co-produits flavoniques peuvent être mis en oeuvre dans des produits de soins pour la peau et pour les cheveux en tant qu'actifs facteur vitaminique P, anti radicaux libres, anti-inflammatoires locaux, apaisants, protecteurs, photoprotecteurs anti-UVB et anti-UVA, anti-pollution, anti-toxiques, anti peaux sensibles et inhibiteurs d'enzymes telles que : l'élastase, la hyaluronidase, l'histidine décarboxylase, la phosphodiestérase de l'AMPc, la lipo-oxygénase, la tyrosinase ou analogues.

Ces extraits actifs totaux de Baobab, où les fractions actives purifiées transformées telles que les mucilages, les protéines, les flavonoïdes, les composés minéraux calciques ou analogues, pourront se présenter sous forme anhydre, sous forme de solutés aqueux ou hydroglycoliques, ou encore sous forme galénique à effet prolongé ou à action différée (liposomes, nanosphères, microsphères, microcapsules ou analogues).

Les extraits selon l'invention sont destinés à être incorporés dans les formes cosmétiques et/ou pharmaceutiques les plus diverses, telles que notamment les lotions, les gels, les hydrogels, les émulsions H/E, les émulsions E/H, les micro-émulsions, les produits de soins cutanés, les produits de soins capillaires ou analogues.

En vue de démontrer les effets bénéfiques des extraits de feuilles de Baobab selon l'invention, et plus particulièrement ceux du coproduit de purification des mucilages tant sur le plan cosmétique que biologique, l'inventeur a procédé à différents tests "in tubo" et "in vitro" d'un tel extrait de feuilles (dénommé ci-après 114-1) obtenu au moyen du procédé décrit dans l'exemple 3 précité.

Les buts recherchés, les modes opératoires mis en oeuvre et les résultats obtenus dans le cadre de ces tests sont brièvement exposés dans ce qui suit.

I) Tests anti-radicaux-libres "in tubo"

Les capacités anti-radicaux libres sont évaluées par une batterie de tests recouvrant aussi bien les formes radicalaires initiales que les formes réactives de l'oxygène ($HO°$ et $O_2^{\overline{°}}$) induites in vivo.

Test anti-DPPH :

Le DPPH (diphenylpicryl-hydrazyl) est un radical libre stable et coloré en violet qui est transformé en son leucodérivé par les substances qui captent et neutralisent les radicaux libres (= effet dit "scavenger").

Dans ce test, la densité optique est mesurée à 513 nm.

Résultats (moyenne sur 2 essais) :

| doses en % (p/v) | taux de leucodérivé formé (en % / témoin) | |
|---|---|---|
| témoin | 0 | |
| 114-1 à 0,003 % | 28 | CI50 = 0,0124 % (p/v) |
| 114-1 à 0,03 % | 91 | |
| 114-1 à 0,3 % | 100 | |

Test anti-HO° avec l'acide salicylique :

Les $HO°$ (formés par H2O2 en présence de Fe++ et d'EDTA) sont révélés par l'acide salicylique.

L'acide salicylique est hydroxylé par $HO°$ en un composé rougeâtre et le taux d'acide salicylique hydroxylé correspond à la densité optique à 490 nm.

Résultats (moyenne sur 2 essais)

| doses en % (p/v) | taux d'hydroxylation avec EDTA | |
|---|---|---|
| témoin | 100 | |
| 114-1 à 0,03 % | 92 | CI50 = 0,24 % (p/v) |
| 114-1 à 0,3 % | 38 | |

Test anti-HO° avec le désoxyribose :

Les $HO°$ (formés par $H_2O_2$ en présence de Fe++ et d'EDTA) sont révélés par le désoxyribose (cette réaction dite de Fenton est aussi réalisée sans EDTA pour mesurer les capacités à complexer le Fer).

Le désoxyribose est oxydé par $HO°$ en dérivés aldéhydiques dosés par l'acide thiobarbiturique, l'acide thiobarbiturique formant par condensation avec les aldéhydes un composé rose (densité optique mesurée à 532 nm)

Résultats (moyenne sur 2 essais) :

| doses en % (p/v) | aldéhyde formé avec EDTA | | aldéhyde formé sans EDTA | |
|---|---|---|---|---|
| témoin | 100 | | 100 | |
| 114-1 à 0,03 % | 99 | CI50 = 0,33 % (p/v) | 76 | CI50 = 0,16 % (p/v) |
| 114-1 à 0,3 % | 55 | | 21 | |

Tests anti-anions superoxydes $O_2^{\overline{\circ}}$

$O_2^{\overline{\circ}}$ est produit par une enzyme induite durant le stress oxydatif : la xanthine oxydase, qui catabolise les bases puriques (adénine, guanine) en acide urique et $O_2^{\overline{\circ}}$.

Ensuite $O_2^{\overline{\circ}}$ se dismute spontanément (ou par la SOD = superoxyde dismutase) en $H_2O_2$ et $O_2$.

Résultats (moyenne sur 2 essais) :

a) $O_2^{\overline{\circ}}$ révélé en luminescence par le luminol

| doses en % (p/v) | % d'inhibition de la luminescence / témoin | |
|---|---|---|
| témoin | 0 | |
| 114-1 à 0,0003 % | 10 | CI50 = 0,0011 % (p/v) |
| 114-1 à 0,003 % | 67 | |
| 114-1 à 0,03 % | 99 | |

b) $O_2^{\overline{\circ}}$ et $H_2O_2$ révélés par le luminol en présence de micropéroxydase

| doses en % (p/v) | % d'inhibition de la luminescence/témoin | |
|---|---|---|
| témoin | 0 | |
| 114-1 à 0,003 % | 13 | CI50 = 0,0096 % (p/v) |
| 114-1 à 0,03 % | 62 | |

c) $O_2^{\overline{\circ}}$ et $H_2O_2$ révélés par le NBT (sel de tétrazolium) (densité optique mesurée à 540 nm)

| doses en % (p/v) | % d'inhibition de la DO à 540 nm/témoin | |
|---|---|---|
| témoin | 0 | |
| 114-1 à 0,03 % | 21 | CI50 = 0,1298 % (p/v) |
| 114-1 à 0,3 % | 67 | |

II) Cytoprotection anti-UVA sur fibroblastes humains en survie "in vitro"

Les UVA pénètrent jusque dans le derme où ils induisent un stress oxydatif révélé par une lipopéroxydation des membranes cytoplasmiques.

Les lipopéroxydes se fragmentent en malonaldialdéhyde qui va réticuler de nombreuses molécules biologiques comme les protéines (inhibition d'enzymes) et les bases nucléiques (mutagénèse).

Pour réaliser les tests, les fibroblastes sont ensemencés dans un milieur de culture défini avec du sérum de veau foetal et le produit 114-1 (dans le milieu défini avec 2 % de sérum) est ajouté 72 heures après l'ensemencement.

Après une incubation de 48 heures à 37° C et $CO_2$ = 5 %, le milieu de culture est remplacé par une solution saline et, les fibroblastes sont irradiés par une dose d'UVA (15 J/cm$^2$ ; tubes du type MAZDA FLUOR TFWN40).

Dès la fin de l'irradiation, le taux de MDA (malonaldialdéhyde) est dosé dans la solution saline surnageante et le taux de protéines est mesuré dans les fibroblastes.

Le MDA est dosé par la réaction à l'acide thiobarbiturique et les protéines selon la méthode dite de Bradford.

Résultats (en % par rapport au témoin. moyenne de 2 essais. chacun en triple) :

| doses en % (p/v) | MDA | protéines |
|---|---|---|
| témoin non irradié | 0 | 100 |
| témoin irradié (UVA) | 100 | 65 |
| milieu défini + 114-1 à 0,005 % | 60 | 61 |
| milieu défini + 114-1 à 0,010 % | 45 | 59 |

III) Cytoprotection anti-UVB sur kératinocytes humains en survie "in vitro"

Les UVB déclenchent une inflammation (érythème, oedème) par activation d'une enzyme, à savoir la phospholipase A2 ou PLA2, qui décroche l'acide arachidonique des phospholipides de la membrane plasmique.

L'acide arachidonique est le précurseur des prostaglandines qui sont des médiateurs de l'inflammation, les prostaglandines E2 (= PGE2) étant formées par la cyclooxygènase.

Pour réaliser les tests, les kératinocytes sont ensemencés dans un milieu défini avec du sérum de veau foetal et le produit 114-1 (dilué dans une solution saline) est ajouté 72 heures après l'ensemencement.

Aussitôt, les kératinocytes sont irradiés par une dose d'UVB (30 mJ/cm$^2$ - tubes du type DUKE FL40E).

Après une incubation de 1 jour à 37° C, $CO_2$ = 5 %, les taux de PGE2 et de LDH sont mesurés dans le milieu surnageant.

Le nombre de kératinocytes adhérents est déterminé (après trypsination) par un compteur de particules.

Le taux de PGE2 est déterminé par un test ELISA et celui de LDH (lactate-déshydrogénase) par une réaction enzymatique.

Résultats (en % par rapport au témoin, moyenne de 3 essais, chacun en double) :

| doses en % (p/v) | nombre de kératinocytes | taux de LDH relarguée* | taux de PGE2 relarguées* |
|---|---|---|---|
| témoin non irradié | 0,77 (million / puits) | 0 | 0 |
| témoin irradié (UVB) | 0,32 | 100 | 100 |
| milieu défini + 114-1 à 0,005 % | 0,38 | 64 | 82 |
| milieu défini + 114-1 à 0,010 % | 0,68 | 15 | 21 |

* = en % par rapport au témoin irradié (= 100 %) et non-irradié (= 0 %).

Il ressort des résultats qui précèdent que l'extrait de feuilles de Baobab analysé et testé (produit 114-1) présente des capacités significatives à :

- capter et neutraliser les radicaux libres et les formes réactives de l'oxygène (HO° et $O_2^{\overline{°}}$), ledit produit 114-1 agissant, au moins en partie, par capture du Fer ("effet ferriprive") ;
- réduire le taux de lipoperoxydation induit par les UVA sur les fibroblastes humains ;
- réduire le taux de PGE2 et la souffrance cellulaire induits par les UVB sur les kératinocytes humains.

Sur le plan cosmétique, l'analyse sensorielle a permis de constater un net effet restructurant, adoucissant et satinant.

A titre d'exemples non limitatifs de réalisations pratiques de l'invention, on décrira ci-après différents produits ou préparations cosmétiques comprenant un extrait de plante du genre Adansonia, notamment de Baobab.

Exemple 1 :

Un produit cosmétique sous forme de gel hydro-protecteur pour le visage conforme à l'invention pourra, par exemple, présenter une composition pondérale, constituée à partir des fractions ou phases A, B, C, D, E et F suivantes, telles qu'indiquées ci-après.

Fraction A :

- Eau distillée        49,950 %
- Elestab 50 J        0,500 %
- Carrageenan        0,100 %

Fraction B :

- Carbomer        0,300 %
- Eau distillée        31,955 %

Fraction C :

- Propylène glycol        2,000 %
- Diméthicone copolyol        3,000 %

Fraction D :

- Triéthanolamine, 20 % en sol. aq.        2,145 %

Fraction E :

- Kathon CG (Rohm et Haas)        0,050 %

Fraction F :

- Extrait aqueux total déshydraté de feuilles d'Adansonia Digitata (extrait type 1)        0,500 %
- Eau distillée        9,500 %

Le procédé de préparation et de fabrication du gel précité consiste essentiellement à préparer séparément les fractions A et B à 75° C sous agitation turbine, puis à les refroidir à température ambiante, à préparer la fraction F par dispersion de l'extrait déshydraté dans 20 fois son poids d'eau, à ajouter à la fraction A successivement les fractions B, C, D, E et F à température ambiante et sous agitation turbine et enfin à poursuivre l'agitation planétaire jusqu'à homogénéisation.

Exemple 2 :

Un produit cosmétique sous forme de crème hydratante pour peaux sensibles et anti-pollution conforme à l'invention pourra, par exemple, présenter une composition pondérale, constituée à partir des fractions ou phases A, B et C suivantes, telles qu'indiquées ci-après.

Fraction A :

- Tegin        10,00 %
- Novata AB        1,00 %
- Miglyol 812        8,00 %
- Cetiol        5,00 %
- Eutanol G        2,00 %

Fraction B :

- Elestab 4112        0,35 %
- Eau distillée        60,65 %
- Glycérine        3,00 %

Fraction C :

- Extrait déshydraté mucilagineux d'Adansonia Digitata (extrait type 1)        1,00%
- Eau distillée        9,00 %

Le procédé de préparation et de fabrication de la crème précitée consiste essentiellement à préparer séparément les fractions A et B à 75° C, à préparer la fraction C, par dispersion sous agitation turbine, à verser la fraction A à 75° C dans la fraction B à 75° C sous agitation turbine, à laisser refroidir le mélange obtenu, sous agitation planétaire jusqu'à 50° C et à introduire la fraction C.

Exemple 3 :

Un produit cosmétique sous forme de crème anti-rides, anti radicaux libres, protectrice du collagène, de l'élastine et de la substance fondamentale, anti-vieillissement cutanée et améliorant la micro-circulation, conforme à l'invention pourra, par exemple, présenter une composition pondérale, constituée à partir des fractions ou phases A, B et C suivantes, telles qu'indiquées ci-après.

Fraction A :

- Cutina CBS       12,00 %
- Cutina E24       2,00 %
- Eumulgin B2       1,00 %
- Eutanol G       3,00 %
- Cétiol SB45       3,00 %
- Cétiol SN       4,00 %

Fraction B :

- Glycérine       5,00 %
- Eau distillée       47,50 %
- Elestab 388       2,50 %
- Vegeseryl HGP       10,00 %

Fraction C :

- Extrait déshydraté flavonoïdique d'Adansonia Digitata (extrait type 3)       2,00 %
- Eau distillée       8,00 %

Le procédé de préparation et de fabrication de la crème précitée consiste essentiellement à préparer séparément les fractions A et B à 75° C, à préparer la fraction C, par dispersion de l'extrait sec dans quatre fois son poids d'eau, à verser la fraction A dans la fraction B sous agitation turbine, à laisser refroidir le mélange obtenu, à ajouter la fraction C vers 50° C et enfin à réaliser une agitation planétaire seule jusqu'à température ambiante.

Exemple 4 :

Un produit cosmétique sous forme de lotion capillaire à vaporiser et photoprotectrice conforme à l'invention pourra, par exemple, présenter une composition pondérale telle qu'indiquée ci-après.

- Ethanol       53,80 %
- Triethanolamine       0,10 %
- Gantrez ES425       3,60 %
- Glycérine       1,00 %
- Extrait flavonoïdique de feuilles d'Adansonia Digitata (extrait type 3)       1,00 %
- Panthénol       0,50 %
- Propane/Butane       40,00 %

Le procédé de préparation et de fabrication de la lotion capillaire précitée consiste essentiellement à mélanger l'ensemble des constituants précités, à filtrer le mélange obtenu et à le conditionner sous propulseur.

Exemple 5

Un produit cosmétique sous forme de lait après-soleil apaisant, réparateur, hydratant, anti-oedème, cicatrisant et radioprotecteur conforme à l'invention pourra, par exemple, présenter une composition pondérale, constituée à partir des tractions ou phases A, B, C et D suivantes, telles qu'indiquées ci-après.

Fraction A :

- Miglyol 812 8,00 %
- Huile de Jojobah 2,00 %
- Acétulan 3,00 %
- Sphingocéryl VEG 1,50 %
- Huile de paraffine 5,00 %
- Brij 76 4,00 %

Fraction B :

- Carbomer 0,50 %
- Elestab 41 12 0,40 %
- Sorbitol 2,00 %
- Uvinul MS40 0,05 %
- Glucam E20 3,00 %
- Eau distillée 58,05 %

Fraction C :

- Triethanolamine en dil. aq. à 20 % 2,50 %

Fraction D :

- Extrait déshydraté total de feuilles d'Adansonia Digitata (extrait type 1 + extrait type 3) 5,00 %
- Eau distillée 5,00 %

Le procédé de préparation et de fabrication du lait après-soleil précité consiste essentiellement à préparer séparément les fractions A et B à 75° C, à verser la fraction A dans la fraction B sous agitation turbine, à ajouter la fraction C, à refroidir, à ajouter la fraction D préalablement homogénéisée vers 50° C et à refroidir le mélange obtenu sous agitation planétaire.

**Revendications**

1. Utilisation d'au moins un extrait de feuilles, fraîches ou séchées, de plante du genre Adansonia appartenant à la famille des Bombacaceaes dans un produit cosmétique à usage topique pour la peau et/ou les phanères, ledit produit comprenant entre 0,50 % et 20,00 % en poids d'un extrait total de feuilles de Baobab.

2. Utilisation selon la revendication 1, **caractérisée en ce que** les feuilles sont extraites par un solvant choisi dans le groupe formé par l'eau, les solutions aqueuses, les alcools et les mélanges de deux ou plusieurs des solvants précités.

3. Utilisation selon l'une quelconque des revendications 1 et 2, **caractérisée en ce que** l'extrait utilisé consiste en un ou des mucilage(s) purifié(s) ou en un extrait riche en mucilage(s) obtenu(s) à partir des feuilles de Baobab.

4. Utilisation selon la revendication 3, **caractérisée en ce que** la purification du ou des mucilage(s) ou de l'extrait riche en mucilage(s) comprend une étape de traitement par une enzyme glycolytique du type β glycosidase.

5. Utilisation selon l'une quelconque des revendications 1 à 4, **caractérisée en ce que** l'extrait utilisé est un co-produit de l'extraction et/ou de la purification de mucilages des feuilles de Baobab, contenant des flavonoïdes, des sels minéraux, des vitamines, des protéines et/ou des composés analogues.

6. Produit cosmétique pour la peau et/ou les phanères, **caractérisé en ce qu'**il consiste en une composition traitante comportant entre 0,50 % et 20,00 % en poids d'un extrait total de feuilles de Baobab.

7. Produit cosmétique pour la peau et/ou les phanères, **caractérisé en ce qu'**il consiste en une composition traitante comportant entre 0,50 % et 20,00 % en poids de mucilages ou d'un extrait riche en mucilages obtenu(s) à partir de feuilles de Baobab.

8. Produit cosmétique pour la peau et/ou les phanères, **caractérisé en ce qu'**il consiste en une composition traitante comportant entre 0,50 % et 20,00 % en poids d'un co-produit de l'extraction et/ou de la purification de mucilages obtenus à partir de feuilles de Baobab.

9. Produit cosmétique selon l'une quelconque des revendications 6 à 8, **caractérisé en ce qu'**il présente une activité de protection contre les UVA et les UVB.

**Patentansprüche**

1. Verwendung mindestens eines Extrakts aus frischen oder getrockneten Blättern einer Pflanze der zur Familie der Bombacaceen zählenden Gattung Adansonia in einem Kosmetikprodukt zur topischen Verwendung für die Haut und/oder die Epithelanhanggebilde, wobei dieses Produkt zwischen 0,50 Gew.-% und 20,00 Gew.-% eines Gesamtextrakts aus Blättern von Adansonia digitata oder Affenbrotbaum enthält.

2. Verwendung nach Anspruch 1, **dadurch gekennzeichnet, daß** die Blätter mit einem Lösungsmittel aus der Gruppe Wasser, wäßrige Lösungen, Alkohole oder Mischungen aus mindestens zwei der genannten Lösungsmittel extrahiert werden.

3. Verwendung nach einem der Ansprüche 1 und 2, **dadurch gekennzeichnet, daß** der verwendete Extrakt aus einem oder mehreren gereinigten Schleimstoff(en) oder einem aus Blättern des Affenbrotbaums gewonnenen schleimstofffreichen Extrakts besteht.

4. Verwendung nach Anspruch 3, **dadurch gekennzeichnet, daß** die Reinigung des Schleimstoffs bzw. der Schleimstoffe oder des schleimstofffreichen Extrakts einen Behandlungsschritt mit einem β-glycosidaseartigen glykolytischen Enzym umfaßt.

5. Verwendung nach einem der Ansprüche 1 bis 4, **dadurch gekennzeichnet, daß** es sich bei dem verwendeten Extrakt um ein Nebenprodukt der Extraktion und/oder Reinigung von Schleimstoffen aus Blättern des Affenbrotbaums handelt, der Flavonoide, Mineralsalze, Vitamine, Proteine und/oder ähnliche Verbindungen enthält.

6. Kosmetikprodukt für die Haut und/oder das Epithelanhanggebilde, **dadurch gekennzeichnet, daß** es aus einer Pflegezusammensetzung mit 0,50 Gew.-% bis 20,00 Gew.-% eines Gesamtextrakts von Affenbrotbaumblättern besteht.

7. Kosmetikprodukt für die Haut und/oder das Epithelanhanggebilde, **dadurch gekennzeichnet, daß** es aus einer Pflegezusammensetzung mit 0,50 Gew.-% bis 20,00 Gew.-% Schleimstoffen oder eines schleimstofffreichen Extrakts aus Blättern des Affenbrotbaums besteht.

8. Kosmetikprodukt für die Haut und/oder das Epithelanhanggebilde, **dadurch gekennzeichnet, daß** es aus einer Pflegezusammensetzung mit 0,50 Gew.-% bis 20,00 Gew.-% eines Nebenprodukts der Extraktion und/oder Reinigung von Schleimstoffen aus Blättern des Affenbrotbaums besteht.

9. Kosmetikprodukt nach einem der Ansprüche 6 bis 8, **dadurch gekennzeichnet, daß** es eine Schutzwirkung gegen die UVA und die UVB aufweist.

**Claims**

1. Use of at least one extract of fresh or dried leaves of a plant of the genus Adansonia, belonging to the Bombacaceaes family, in a cosmetic product for topical use on the skin and/or skin appendages, said product comprising between 0.50% and 20.00% by weight of a total extract of Baobab leaves.

2. Use according to claim 1, **characterised in that** the leaves are extracted by a solvent selected from the group consisting of water, aqueous solutions, alcohols, and mixtures of two or more of the aforementioned solvents.

3. Use according to any one claims 1 and 2, **characterised in that** the extract that is used consists of one or more purified mucilage(s) or of an extract rich in mucilage(s) obtained from Baobab leaves.

4. Use according to claim 3, **characterised in that** the purification of the mucilage(s) or of the extract rich in mucilage (s) comprises a step of treatment with a glycolytic enzyme of the β glycosidase type.

5. Use according to any one of claims 1 to 4, **characterised in that** the extract that is used is a co-product of the extraction and/or of the purification of mucilages of Baobab leaves, containing flavonoids, mineral salts, vitamins, proteins and/or similar compounds.

6. Cosmetic product for the skin and/or skin appendages, **characterised in that** it consists of a treatment composition comprising between 0.50% and 20.00% by weight of a total extract of Baobab leaves.

7. Cosmetic product for the skin and/or skin appendages, **characterised in that** it consists of a treatment composition comprising between 0.50% and 20.00% by weight of mucilages or of an extract rich in mucilage(s) obtained from Baobab leaves.

8. Cosmetic product for the skin and/or skin appendages, **characterised in that** it consists of a treatment composition comprising between 0.50% and 20.00% by weight of a co-product of the extraction and/or the purification of mucilages obtained from Baobab leaves.

9. Cosmetic product according to any one of claims 6 to 8, **characterised in that** it exhibits a protective activity against UVA and UVB.